(19) Europäisches Patentamt
European Patent Office
Office européen des brevets

(11) EP 1 506 711 A2

(12) # EUROPÄISCHE PATENTANMELDUNG

(43) Veröffentlichungstag:
**16.02.2005 Patentblatt 2005/07**

(51) Int Cl.[7]: **A01N 43/54**
// (A01N43/54, 61:00)

(21) Anmeldenummer: **04024463.4**

(22) Anmeldetag: **08.11.1999**

(84) Benannte Vertragsstaaten:
**AT BE CH CY DE DK ES FI FR GB GR IE IT LI LU MC NL PT SE**

(30) Priorität: **20.11.1998 DE 19853559**
**23.08.1999 DE 19939841**

(62) Dokumentnummer(n) der früheren Anmeldung(en) nach Art. 76 EPÜ:
**99953975.2 / 1 130 963**

(71) Anmelder: **Bayer CropScience AG**
**40789 Monheim (DE)**

(72) Erfinder:
- **Mauler-Machnik, Astrid, Dr.**
  **42799 Leichlingen (DE)**
- **Wachendorff-Neumann, Ulrike, Dr.**
  **56566 Neuwied (DE)**
- **Gayer, Herbert, Dr.**
  **40789 Monheim (DE)**

Bemerkungen:
Diese Anmeldung ist am 14/10/2004 als Teilanmeldung zu der unter INID-Kode 62 erwähnten Anmeldung eingereicht worden.

(54) **Fungizide Wirkstoffkombinationen**

(57) Beschrieben werden neue Wirkstoffkombinationen aus Verbindungen der Formel (I)

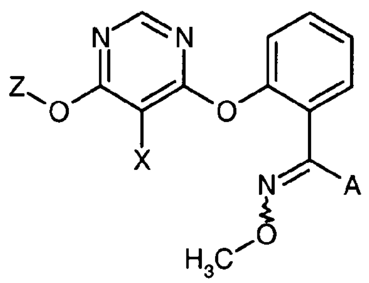

in welcher
Z, X und A die in der Beschreibung angegebene Bedeutung haben,
mit bekannten Wirkstoffen sowie deren Verwendung zur Bekämpfung von phytopathogenen Pilzen.

EP 1 506 711 A2

## Beschreibung

Die vorliegende Anmeldung betrifft neue Wirkstoffkombinationen, die aus Pyrimidin-Derivaten einerseits und weiteren bekannten fungiziden Wirkstoffen andererseits bestehen und sehr gut zur Bekämpfung von phytopathogenen Pilzen geeignet sind.

Es ist bereits bekannt, daß Pyrimidin-Derivate fungizide Eigenschaften besitzt (vgl. DE-A-19 646 407). Die Wirksamkeit dieses Stoffes ist gut; sie läßt jedoch bei niedrigen Aufwandmengen in manchen Fällen zu wünschen übrig.

Ferner ist schon bekannt, daß zahlreiche Azol-Derivate, aromatische Carbonsäure-Derivate, Morpholin-Verbindungen und andere Heterocyclen zur Bekämpfung von Pilzen eingesetzt werden können (vgl. K.H. Büchel "Pflanzenschutz und Schädlingsbekämpfung" Seiten 87, 136, 140, 141 und 146 bis 153, Georg Thieme Verlag, Stuttgart 1977). Die Wirkung der betreffenden Stoffe ist aber bei niedrigen Aufwandmengen nicht immer befriedigend.

Es wurde nun gefunden, daß die neuen Wirkstoffkombinationen aus Verbindungen der allgemeinen Formel (I)

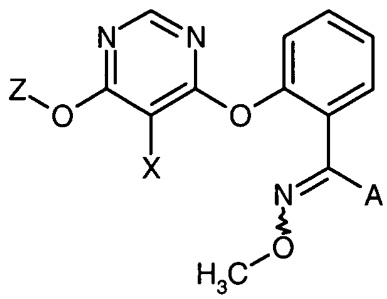

in welcher

Z für gegebenenfalls substituiertes Phenyl steht,
X für Halogen steht und
A für Heterocyclyl, $-COOCH_3$ oder $-CO-NH-CH_3$ steht und

jeweils einer der nachfolgenden Verbindungen

| | |
|---|---|
| 1) | Spiroxamin |
| 2) | Quinoxyfen (DE 795) |
| 3) | Tebuconazole |
| 4) | Fenpropidin |
| 5) | Fenpropimorph |
| 6) | N-( 1-Cyano-1,2-dimethylpropyl)-2-(2,4-dichlorophenoxy)-propionamid (R,S)- und (R,R)- und (S, R)- und (S, S) |
| 7) | Chlorothalonil (DAC 2787) |
| 8) | Triadimefon |
| 9) | Triadimenol |
| 10) | Epoxiconazole |
| 11) | Metconazole |
| 12) | Fluquinconazole |
| 13) | Cyproconazole |
| 14) | Penconazole |
| 15) | Kresoximmethyl |

(fortgesetzt)

| | |
|---|---|
| 16) | Azoxystrobin |
| 17) | Cyprodinil |
| 18) | Iminoctadien-triacetat (Befran) |
| 19) | Flusilazole (Harvesan) |
| 20) | Prochloraz (Sportak) |
| 21) | Propiconazole (Desmel) |
| 22) | Bitertanol (KWG 0599) |
| 23) | Imidacloprid (NTN 33893) |
| 24) | Dichlofluanid (Euparen) |
| 25) | Tolylfluanid (Euparen M) |
| 26) | Metalaxyl (Ridomil) |
| 27) | Fenpiclonil |
| 28) | Difenoconazole |
| 29) | Fludioxonil |
| 30) | Carbendazim, Benomyl |
| 31) | Fuberidazol |
| 32) | Imazalil |
| 33) | Triazoxide (SAS 9244) |
| 34) | Cyfluthrin (Pyrethroid) |
| 35) | Guazatine |
| 36) | Acibenzolar-S-methyl (Bion) |
| 37) | Pencycuron (Monceren) |
| 38) | Flutolanil (Moncut) |
| 39) | Tricyclazole (Beam) |
| 40) | Propineb (Antracol) |
| 41) | Procymidone (Sumisclex) |
| 42) | Mancozeb |
| 43) | Folpet (Phaltan) |
| 44) | Dimetomorph |
| 45) | Cymoxanil (Curzate) |
| 46) | Fosetyl-Al (Aliette) |
| 47) | Famoxadone |
| 48) | Pyrimethanil |
| 49) | Mepanipyrim |
| 50) | Iprovalicarb |
| 51) | Fenhexamid |
| 52) | Carpropamid |
| 53) | Fluazinam |
| 54) | Captan |

(fortgesetzt)

| | |
|---|---|
| 55) | Chinomethionat (Morestan) |
| 56) | Fenamidone (RP 7213) |
| 57) | Clothianidin |
| 58) | Thiacloprid |
| 59) | Diacloden |
| 60) | Acetamiprid |
| 61) | MTI 334 |
| 62) | Schwefel |
| 63) | Kupfer |
| 64) | Rovral |
| 65) | Ronilan |
| 66) | Rabcide |
| 67) | Hinosan |
| 68) | Coratop |
| 69) | 2-(1-Chlor-cyclopropyl)-1-(2-chlorphenyl)-3-(5-mercapto-1,2,4-triazol-1-yl)-propan-2-ol |
| 70) | 1-(3,5-Dimethylisoxazol-4-sulfonyl)-2-chlor-6,6-difluor-[1,3]-dioxolo-[4,5f]benzimidazol |
| 71) | 3-{1-[4-<2-Chlorphenoxy>-5-fluorpyrimid-6-yloxy)-phenyl]-1-(methoximino)-methyl}-5,6-dihydro-1,4,2-dioxazin |
| 72) | Zoxamide |
| 73) | Cyamidazosulfamid |
| 74) | Silthiopham |
| 75) | Trifloxystrobin |
| 76) | N-Methyl-2-(methoxyimino)-2-[2-([1-(3-trifluoromethyl-phenyl)ethoxy]iminomethyl)phenyl]acetamid |
| 77) | 2-[2-([2-Phenyl-2-methoxyimino-1-methylethyl]iminooxymethyl)phenyl]-2-methoxyimino-N-methylacetamid |
| 78) | 2-[2-([2-(4-Fluorophenyl)-2-methoxyimino-1-methylethyl]-iminooxymethyl)phenyl]-2-methoxyimino-N-methylacetamid |
| 79) | 2-[4-Methoxy-3-(1-methylethoxy)-1,4-diazabuta-1,3-dienyloxymethyl]phenyl-2-methoximino-N-methylacetamid |
| 80) | Methyl N-(2-[1-(4-chlorophenyl)pyrazol-3-yloxymethyl]-phenyl)-N-methoxycarbamat |
| 81) | 2,4-Dihydro-5-methoxy-2-methyl-4-[2-([([1-(3-trifluoromethylphenyl)ethylidene]amino)oxy]methyl)phenyl]-3H-1,2,4-triazol-3-one |
| 82) | Picoxystrobin |

in einem Mischungsverhältnis einer Verbindung der Formel (I) zu jeweils einer Verbindung der Formeln 1) bis 82) von 20:1 bis 1:50 Gewichtsteilen sehr gute fungizide Eigenschaften besitzen.

Überraschenderweise ist die fungizide Wirkung der erfindungsgemäßen Wirkstoffkombination wesentlich höher als die Summe der Wirkungen der einzelnen Wirkstoffe. Es liegt ein nicht vorhersehbarer echter synergistischer Effekt vor und nicht nur eine Wirkungsergänzung.

Bevorzugt genannt seien Verbindungen der Formel (I), in welcher

Z für eine Gruppierung

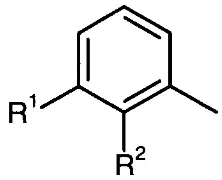

steht, worin

$R^1$ und $R^2$ unabhängig voneinander für Wasserstoff, Methyl, Ethyl, Methoxy, Ethoxy, Chlor, Brom, Fluor oder Cyano stehen,

X für Fluor steht und

A für $-CO-NH-CH_3$ steht,

genannt.

Insbesondere seien die Verbindungen der Formel (I), in welcher

$R^1$ für Wasserstoff steht und

$R^2$ für Methyl, Ethyl, Methoxy, Chlor, Brom, Fluor oder Cyano steht,

sowie Verbindungen der Formel (I), in denen

$R^1$ für Methyl steht und

$R^2$ für Wasserstoff, Methyl, Ethyl, Methoxy, Chlor, Brom, Fluor oder Cyano steht,

sowie Verbindungen der Formel (I), in denen

$R^2$ für Methyl steht und

$R^1$ für Wasserstoff, Methyl, Ethyl, Methoxy, Chlor, Brom, Fluor oder Cyano steht,

im einzelnen genannt.

Die Wirkstoffe der Formel (I) sind bekannt (vergl. z. B. DE-A 19 646 407, WO 97-27189 oder GB 2253624).

Die in den erfindungsgemäßen Kombinationen außerdem vorhandenen Wirkstoffe sind ebenfalls bekannt. Die Wirkstoffe werden z.B. in The Pepticide Manual, 11th Edition, British Crop Protection Council (BCPC) beschrieben.

Die erfindungsgemäßen Wirkstoffkombinationen enthalten neben mindestens einem Wirkstoff der Formel (I) mindestens einen Wirkstoff von den Verbindungen der Gruppen 1) bis 83). Sie können darüber hinaus auch weitere fungizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im allgemeinen enthalten die erfindungsgemäßen Kombinationen den Wirkstoff der Formel (I) und den Mischpartner in den in der nachfolgenden Tabelle angegebenen bevorzugten und besonders bevorzugten Mischungsverhältnissen:

**[0012]** * die Mischungsverhältnisse basieren auf Gewichtsverhältnissen. Das Verhältnis ist zu verstehen als Wirkstoff der Formel I: Mischpartner

| **Mischpartner** | **bevorzugtes Mischungs-verhältnis*** | **besonders bevorzugtes Mischungs- verhältnis*** |
|---|---|---|
| Spiroxamin | 10:1 bis 1:20 | 5:1 bis 1:10 |
| Quinoxyfen (DE 795) | 10:1 bis 1:20 | 5:1 bis 1:10 |
| Tebuconazole | 10:1 bis 1:10 | 5:1 bis 1:5 |

(fortgesetzt)

| Mischpartner | bevorzugtes Mischungs- verhältnis* | besonders bevorzugtes Mischungs- verhältnis* |
|---|---|---|
| Fenpropidin | 10:1 bis 1:20 | 5:1 bis 1:10 |
| Fenpropimorph | 10:1 bis 1:20 | 5:1 bis 1:10 |
| N-(1-Cyano-1,2-dimethylpropyl)-2-(2,4-dichlorophenoxy) propionamid (R,S)- und (R,R)- und (S, R)- und (S,S) | 10:1 bis 1:10 | 5:1 bis 1:5 |
| Chlorothalonil (DAC 2787) | 1:1 bis 1:50 | 1:5 bis 1:20 |
| Triadimefon | 10:1 bis 1:10 | 5:1 bis 1:5 |
| Triadimenol | 10:1 bis 1:10 | 5:1 bis 1:5 |
| Epoxiconazole | 10:1 bis 1:10 | 5:1 bis 1:5 |
| Metconazole | 10:1 bis 1:10 | 5:1 bis 1:5 |
| Fluquinconazole | 10:1 bis 1:10 | 5:1 bis 1:5 |
| Cyproconazole | 10:1 bis 1:10 | 5:1 bis 1:5 |
| Penconazole | 10:1 bis 1:10 | 5:1 bis 1:5 |
| Kresoximmethyl | 10:1 bis 1:10 | 5:1 bis 1:5 |
| Azoxystrobin | 10:1 bis 1:10 | 5:1 bis 1:5 |
| Cyprodinil | 5:1 bis 1:20 | 1:1 bis 1:10 |
| Iminoctadien-triacetat (Befran) | 10:1 bis 1:10 | 5:1 bis 1:5 |
| Flusilazole (Harvesan) | 10:1 bis 1:10 | 5:1 bis 1:5 |
| Prochloraz (Sportak) | 10:1 bis 1:10 | 5:1 bis 1:5 |
| Propiconazole (Desmel) | 10:1 bis 1:10 | 5:1 bis 1:5 |
| Bitertanol | 10:1 bis 1:10 | 5:1 bis 1:5 |
| Imidacloprid | 20:1 bis 1:20 | 10:1 bis 1:10 |
| Dichlofluanid (Euparen) | 1:1 bis 1:50 | 1:1 bis 1:20 |
| Tolylfluanid (Euparen M) | 1:1 bis 1:50 | 1:1 bis 1:20 |
| Metalaxyl (Ridomil) | 10:1 bis 1:10 | 5:1 bis 1:5 |
| Fenpiclonil | 10:1 bis 1:10 | 5:1 bis 1:5 |
| Difenoconazole | 10:1 bis 1:10 | 5:1 bis 1:5 |
| Fludioxonil | 10:1 bis 1:10 | 5:1 bis 1:5 |
| Carbendazim,Benomyl | 10:1 bis 1:10 | 5:1 bis 1:5 |
| Fuberidazol | 20:1 bis 1:10 | 10:1 bis 1:5 |
| Imazalil | 20:1 bis 1:10 | 10:1 bis 1:5 |
| Triazoxide (SAS 9244) | 20:1 bis 1:10 | 10:1 bis 1:5 |
| Cyfluthrin (Pyrethroid) | 20:1 bis 1:20 | 10:1 bis 1:10 |
| Guazatine | 10:1 bis 1:10 | 5:1 bis 1:5 |
| Acibenzolar-S-methyl (Bion) | 50:1 bis 1:50 | 20:1 bis 1:10 |
| Pencycuron (Monceren) | 10:1 bis 1:10 | 5:1 bis 1:5 |
| Flutolanil (Moncut) | 10:1 bis 1:10 | 5:1 bis 1:5 |

(fortgesetzt)

| Mischpartner | bevorzugtes Mischungs- verhältnis* | besonders bevorzugtes Mischungs- verhältnis* |
|---|---|---|
| Tricyclazole (Beam) | 10:1 bis 1:10 | 5:1 bis 1:5 |
| Propineb (Antracol) | 1:1 bis 1:50 | 1:5 bis 1:20 |
| Procymidone (Sumisclex) | 10:1 bis 1:10 | 5:1 bis 1:5 |
| Mancozeb | 1:1 bis 1:50 | 1:5 bis 1:20 |
| Folpet (Phaltan) | 1:1 bis 1:50 | 1:5 bis 1:20 |
| Dimetomorph | 10:1 bis 1:10 | 5:1 bis 1:5 |
| Cymoxanil (Curzate) | 10:1 bis 1:10 | 5:1 bis 1:5 |
| Fosetyl-Al (Aliette) | 10:1 bis 1:50 | 1:1 bis 1:10 |
| Famoxadone | 10:1 bis 1:10 | 5:1 bis 1:5 |
| Pyrimethanil | 5:1 bis 1:20 | 1:1 bis 1:10 |
| Mepanipyrim | 5:1 bis 1:20 | 1:1 bis 1:10 |
| Iprovalicarb | 10:1 bis 1:10 | 5:1 bis 1:5 |
| Fenhexamid | 10:1 bis 1:10 | 5:1 bis 1:5 |
| Carpropamid | 10:1 bis 1:10 | 5:1 bis 1:5 |
| Fluazinam | 10:1 bis 1:10 | 5:1 bis 1:5 |
| Captan | 5:1 bis 1:50 | 1:1 bis 1:20 |
| Chinomethionat (Morestan) | 5:1 bis 1:50 | 1:1 bis 1:20 |
| Fenamidone (RP 7213) | 10:1 bis 1:10 | 5:1 bis 1:5 |
| Clothianidin | 20:1 bis 1:20 | 10:1 bis 1:10 |
| Thiacloprid | 20:1 bis 1:20 | 10:1 bis 1:10 |
| Diacloden | 20:1 bis 1:20 | 10:1 bis 1:10 |
| Acetamiprid | 20:1 bis 1:20 | 10:1 bis 1:10 |
| MTI 334 | 20:1 bis 1:20 | 10:1 bis 1:10 |
| Schwefel | 20:1 bis 1:20 | 10:1 bis 1:10 |
| Kupfer | 20:1 bis 1:20 | 10:1 bis 1:10 |
| Rovral | 10:1 bis 1:10 | 5:1 bis 1:5 |
| Ronilan | 10:1 bis 1:10 | 5:1 bis 1:5 |
| Rabcide | 10:1 bis 1:10 | 5:1 bis 1:5 |
| Hinosan | 10:1 bis 1:10 | 5:1 bis 1:5 |
| Coratop | 10:1 bis 1:10 | 5:1 bis 1:5 |
| 2-(1-Chlor-cyclopropyl)-1-(2-chlorphenyl)-3-(5-mercapto-1,2,4-triazol-1-yl)-propan-2-ol | 20:1 bis 1:5 | 20:1 bis 1:5 |
| 1-(3,5-Dimethylisoxazol-4-sulfonyl)-2-chlor-6,6-difluor-[1,3]-dioxolo-[4,5f]benzimidazol | 50:1 bis 1:10 | 20:1 bis 1:5 |

(fortgesetzt)

| Mischpartner | bevorzugtes Mischungsverhältnis* | besonders bevorzugtes Mischungs- verhältnis* |
|---|---|---|
| 3-{1-[4-<2-Chlorphenoxy>-5-fluorpyrimid-6-yloxy)-phenyl]-1-(methoximino)-methyl}-5,6-dihydro-1,4,2-dioxazin | 10:1 bis 1:10 | 5:1 bis 1:5 |
| Zoxamide | 50:1 bis 1:10 | 20:1 bis 1:5 |
| Cyamidazosulfamid | 20:1 bis 1:5 | 20:1 bis 1:5 |
| Silthiopham | 20:1 bis 1:10 | 20:1 bis 1:5 |
| Trifloxystrobin | 10:1 bis 1:10 | 5:1 bis 1:5 |
| N-Methyl-2-(methoxyimino)-2-[2-([1-(3-trifluoromethylphenyl)-ethoxy]-iminomethyl)-phenyl]-acetamid | 10:1 bis 1:10 | 5:1 bis 1:5 |
| 2-[2-([2-Phenyl-2-methoxyimino-1-methylethyl]-iminooxymethyl) phenyl]-2-methoxyimino-N-methylacetamid | 10:1 bis 1:10 | 5:1 bis 1:5 |
| 2-[2-([2-(4-Fluorophenyl)-2-methoxyimino-1-methylethyl] iminooxymethyl)phenyl]-2-methoxyimino-N-methylacetamid | 10:1 bis 1:10 | 5:1 bis 1:5 |
| 2-[4-Methoxy-3-(1-methylethoxy)-1,4-diazabuta-1,3-dienyloxymethyl] phenyl-2-methoximino-N-methylacetamid | 10:1 bis 1:10 | 5:1 bis 1:5 |
| Methyl N-(2-[1-(4-chlorophenyl) pyrazol-3-yloxymethyl]phenyl)-N-methoxycarbamat | 10:1 bis 1:10 | 5:1 bis 1:5 |
| 2,4-Dihydro-5-methoxy-2-methyl-4-[2-([([1-(3-trifluoromethyl-phenyl)-ethylidene]-amino)-oxy]-methyl)phenyl]-3H-1,2,4-triazol-3-one | 10:1 bis 1:10 | 5:1 bis 1:5 |
| Picoxystrobin | 10:1 bis 1:10 | 5:1 bis 1:5 |

Die erfindungsgemäßen Wirkstoffkombinationen besitzen sehr gute fungizide Eigenschaften und lassen sich vor allem zur Bekämpfung von phytopathogenen Pilzen, wie Plasmodiophoromycetes, Oomycetes, Chytridiomycetes, Zygomycetes, Ascomycetes, Basidiomycetes, Deuteromycetes usw. einsetzen.

Die gute Pflanzenverträglichkeit der Wirkstoffkombinationen in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffkombinationen können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe bzw. der Wirkstoffkombinationen mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgierund/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe der Formel (I) und die erfindungsgemäßen Wirkstoffkombinationen können in den Formulierungen in Mischung mit anderen Wirkstoffen vorliegen, wie Fungizide, Insektizide, Akarizide und Herbizide, sowie in Mischungen mit Düngemitteln oder Pflanzenwachstumsregulatoren.

Für solche Mischungen kommen beispielsweise infrage:

**Fungizide:**

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyanophenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox, Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung, Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Validamycin A, Vinclozolin,
Zineb, Ziram

**Bakterizide:**

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184 699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin, Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301/5302, Zetamethrin.

Die Wirkstoffkombinationen können als solche, in Form ihrer Formulierungen oder den daraus bereiteten Anwendungsformen, wie gebrauchsfertige Lösungen, emulgierbare Konzentrate, Emulsionen, Suspensionen, Spritzpulver, lösliche Pulver und Granulate, angewendet werden.Die Anwendung geschieht in üblicher Weise, z.B. durch Gießen, Verspritzen, Versprühen, Verstreuen, Verstreichen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die gute fungizide Wirkung der erfindungsgemäßen Wirkstoffkombinationen geht aus den nachfolgenden Beispielen hervor. Während die einzelnen Wirkstoffe in der fungiziden Wirkung Schwächen aufweisen, zeigen die Kombinationen eine Wirkung, die über eine einfache Wirkungssummierung hinausgeht.

Ein synergistischer Effekt liegt bei Fungiziden immer dann vor, wenn die fungizide Wirkung der Wirkstoffkombinationen größer ist als die Summe der Wirkungen der einzeln applizierten Wirkstoffe.

Die zu erwartende Wirkung für eine gegebene Kombination zweier Wirkstoffe kann (vgl. Colby, S.R., "Calculating Synergistic and Antagonistic Responses of Herbicide Combinations", Weeds 15, Seiten 20-22, 1967) wie folgt berechnet werden:
Wenn

X den Wirkungsgrad, ausgedrückt in % der unbehandelten Kontrolle, beim Einsatz des Wirkstoffes A in einer Konzentration von m ppm,

Y den Wirkungsgrad, ausgedrückt in % der unbehandelten Kontrolle, beim Einsatz des Wirkstoffes B in einer Konzentration von m ppm,

E den erwarteten Wirkungsgrad, ausgedrückt in % der unbehandelten Kontrolle, beim Einsatz des Wirkstoffes A und B in einer Konzentrationen von m und n ppm bedeutet,

$$\text{dann ist } E = X + Y - \frac{X \cdot N}{100}\ .$$

Ist die tatsächliche fungizide Wirkung größer als berechnet, so ist die Kombination in ihrer Wirkung überadditiv, d.h. es liegt ein synergistischer Effekt vor. In diesem Fall muß der tatsächlich beobachtete Wirkungsgrad größer sein als der aus der oben angeführten Formel errechnete Wert für den erwarteten Wirkungsgrad (E).

## Patentansprüche

**1.** Wirkstoffkombination enthaltend eine Verbindung der Formel (I),

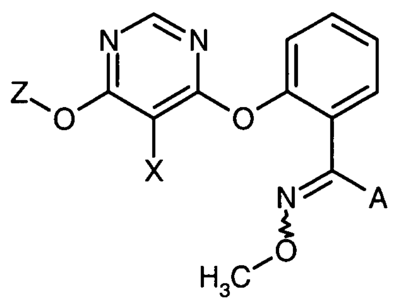

in welcher

Z für gegebenenfalls substituiertes Phenyl steht,
X für Halogen steht und
A für Heterocyclyl, $-COOCH_3$ oder $-CO-NH-CH_3$ steht und

jeweils einer der nachfolgenden Verbindungen

| | |
|---|---|
| 2) | Quinoxyfen (DE 795) |
| 3) | Tebuconazole |
| 4) | Fenpropidin |
| 5) | Fenpropimorph |
| 6) | N-(1-Cyano-1,2-dimethylpropyl)-2-(2,4-dichlorophenoxy)-propionamid (R,S)- und (R,R)- und (S, R)- und (S, S) |

(fortgesetzt)

| | |
|---|---|
| 7) | Chlorothalonil (DAC 2787) |
| 8) | Triadimefon |
| 9) | Triadimenol |
| 10) | Epoxiconazole |
| 11) | Metconazole |
| 12) | Fluquinconazole |
| 13) | Cyproconazole |
| 14) | Penconazole |
| 15) | Kresoximmethyl |
| 16) | Azoxystrobin |
| 17) | Cyprodinil |
| 18) | Iminoctadien-triacetat (Befran) |
| 19) | Flusilazole (Harvesan) |
| 20) | Prochloraz (Sportak) |
| 21) | Propiconazole (Desmel) |
| 22) | Bitertanol (KWG 0599) |
| 23) | Imidacloprid (NTN 33893) |
| 24) | Dichlofluanid (Euparen) |
| 25) | Tolylfluanid (Euparen M) |
| 26) | Metalaxyl (Ridomil) |
| 27) | Fenpiclonil |
| 28) | Difenoconazole |
| 29) | Fludioxonil |
| 30) | Carbendazim, Benomyl |
| 31) | Fuberidazol |
| 32) | Imazalil |
| 33) | Triazoxide (SAS 9244) |
| 34) | Cyfluthrin (Pyrethroid) |
| 35) | Guazatine |
| 36) | Acibenzolar-S-methyl (Bion) |
| 37) | Pencycuron (Monceren) |
| 38) | Flutolanil (Moncut) |
| 39) | Tricyclazole (Beam) |
| 40) | Propineb (Antracol) |
| 41) | Procymidone (Sumisclex) |
| 42) | Mancozeb |
| 43) | Folpet (Phaltan) |
| 44) | Dimetomorph |
| 45) | Cymoxanil (Curzate) |

(fortgesetzt)

| | |
|---|---|
| 46) | Fosetyl-Al (Aliette) |
| 47) | Famoxadone |
| 48) | Pyrimethanil |
| 49) | Mepanipyrim |
| 50) | Iprovalicarb |
| 51) | Fenhexamid |
| 52) | Carpropamid |
| 53) | Fluazinam |
| 54) | Captan |
| 55) | Chinomethionat (Morestan) |
| 56) | Fenamidone (RP 7213) |
| 57) | Clothianidin |
| 58) | Thiacloprid |
| 59) | Diacloden |
| 60) | Acetamiprid |
| 61) | MTI 334 |
| 62) | Schwefel |
| 63) | Kupfer |
| 64) | Rovral |
| 65) | Ronilan |
| 66) | Rabcide |
| 67) | Hinosan |
| 68) | Coratop |
| 69) | 2-(1-Chlor-cyclopropyl)-1-(2-chlorphenyl)-3-(5-mercapto-1,2,4-triazol-1-yl)-propan-2-ol |
| 70) | 1-(3,5-Dimethylisoxazol-4-sulfonyl)-2-chlor-6,6-difluor-[1,3]-dioxolo-[4,5f]benzimidazol |
| 71) | 3-{1-[4-<2-Chlorphenoxy>-5-fluorpyrimid-6-yloxy)-phenyl]-1-(methoximino)-methyl }-5,6-dihydro-1,4,2-dioxazin |
| 72) | Zoxamide |
| 73) | Cyamidazosulfamid |
| 74) | Silthiopham |
| 75) | Trifloxystrobin |
| 76) | N-Methyl-2-(methoxyimino)-2-[2-([1-(3-trifluoromethylphenyl)-ethoxy]iminomethyl)phenyl]acetamid |
| 77) | 2-[2-([2-Phenyl-2-methoxyimino-1-methylethyl]iminooxymethyl)phenyl]-2-methoxyimino-N-methylacetamid |
| 78) | 2-[2-([2-(4-Fluorophenyl)-2-methoxyimino-1-methylethyl]-iminooxymethyl)phenyl]-2-methoxyimino-N-methylacetamid |
| 79) | 2-[4-Methoxy-3-(1-methylethoxy)-1,4-diazabuta-1,3-dienyloxymethyl]phenyl-2-methoximino-N-methylacetamid |
| 80) | Methyl N-(2-[1-(4-chlorophenyl)pyrazol-3-yloxymethyl]phenyl)-N-methoxycarbamat |
| 81) | 2,4-Dihydro-5-methoxy-2-methyl-4-[2-([([1-(3-trifluoromethyl-phenyl)ethylidene]amino)oxy]methyl)phenyl]-3H-1,2,4-triazol-3-one |

(fortgesetzt)

| | |
|---|---|
| 82) | Picoxystrobin |

in einem Mischungsverhältnis einer Verbindung der Formel (I) zu jeweils einer Verbindung der Formeln 1) bis 82) von 20:1 bis 1:50 Gewichtsteilen sehr gute fungizide Eigenschaften besitzen.

2. Mittel, **gekennzeichnet durch** einen Gehalt an einer Wirkstoffkombination wie in Anspruch 1 definiert.

3. Verfahren zur Bekämpfung von Pilzen, **dadurch gekennzeichnet, daß** man Wirkstoffkombinationen wie in Anspruch 1 bzw. Mittel wie in Anspruch 2 definiert auf die Pilze und/oder deren Lebensraum einwirken läßt.

4. Verwendung von Wirkstoffkombinationen wie in Anspruch 1 definiert zur Bekämpfung von Pilzen.

5. Verfahren zur Herstellung von fungiziden Mitteln, **dadurch gekennzeichnet, daß** man Wirkstoffkombinationen wie in Anspruch 1 definiert mit Streckmitteln und/oder oberfächenaktiven Stoffen vermischt.